# EUROPÄISCHE PATENTSCHRIFT

(11) **EP 3 752 040 B1**
(45) Veröffentlichungstag und Bekanntmachung des Hinweises auf die Patenterteilung: **09.03.2022**
(21) Anmeldenummer: 19705452.1
(22) Anmeldetag: 23.01.2019
(51) Int. Cl.: A61B 1/00, A61B 1/015

(54) **VENTILSCHIEBER FÜR EINE ALS INSUFFLATIONS- UND SPÜLVENTIL DIENENDE VENTILEINHEIT, VENTILEINHEIT MIT EINEM ENTSPRECHENDEN VENTILSCHIEBER SOWIE VERFAHREN ZUR HERSTELLUNG EINES VENTILSCHIEBERS**
VALVE SLIDE FOR A VALVE UNIT USED AS AN INSUFFLATION AND PURGING VALVE, VALVE UNIT HAVING A CORRESPONDING VALVE SLIDE, AND METHOD FOR PRODUCING A VALVE SLIDE
TIROIR DE SOUPAPE DESTINÉ À UNE UNITÉ DE SOUPAPE SERVANT DE SOUPAPE D'INSUFFLATION ET DE VIDANGE, UNITÉ DE SOUPAPE DOTÉE D'UN TIROIR DE SOUPAPE CORRESPONDANT AINSI QUE PROCÉDÉ DE FABRICATION D'UN TIROIR DE SOUPAPE

(30) Priorität: 14.02.2018 DE 102018103355
(43) Veröffentlichungstag der Anmeldung: 23.12.2020
(73) Patentinhaber: FUJIFILM MEDWORK GMBH, 91315 Höchstadt/Aisch (DE)
(72) Erfinder: STIRNWEISS, Matthias, 91475 Lonnerstadt (DE); FISCHER, Gerald, 91315 Höchstadt/Aisch (DE)
(74) Vertreter: Gosdin, Carstensen & Partner Patentanwälte Partnerschaftgesellschaft mbB
(86) Internationale Anmeldenummer: PCT/DE2019/100071
(87) Internationale Veröffentlichungsnummer: WO 2019/158149

(56) Entgegenhaltungen:
- EP-A1- 3 073 889
- EP-A1- 3 108 799
- EP-B1- 3 073 889
- US-A1- 2016 309 987
- US-A1- 2017 143 194

## Beschreibung

### Gebiet der Erfindung

Die Erfindung betrifft einen Ventilschieber für eine als Insufflations- und Spülventil dienende Ventileinheit eines medizinischen Endoskops, der mit einem distalen Steuerungsabschnitt in eine mit Luftzufuhr- und Luftaustrittskanälen sowie Spülflüssigkeitszufuhr- und Spülflüssigkeitsaustrittskanälen verbundene, zumindest mittelbar in einem Handbetätigungsteil des Endoskops ausgebildete Aufnahmebohrung einsetzbar ist, wobei der Ventilschieber einen proximalen Betätigungsabschnitt aufweist, der durch einen Haltering hindurchgeführt sowie mit einem Betätigungsknopf versehen ist, wobei der Betätigungsknopf über eine Druckfeder einerseits am Haltering abgestützt ist und andererseits ein ringförmiger Vorsprung des Ventilschiebers als in axialer Richtung wirkender Anschlag mit dem Haltering zusammenwirkt, und wobei am distalen Steuerungsabschnitt des Ventilschiebers Dichtringe vorgesehen sind, die radial über den Ventilschieber vorstehen und vorgesehen sind, um, gleitend an einer Innenmantelfläche der Aufnahmebohrung geführt, zumindest einen Steuerraum der Ventileinheit abzudichten.

Weiterhin betrifft die Erfindung auch eine Ventileinheit eines medizinischen Endoskops, die als Insufflationsventil und Spülventil mit einem gemeinsamen Ventilschieber dient, der mit einem distalen Steuerungsabschnitt verschiebbar in einer Aufnahmebohrung eines Ventilgehäuses angeordnet ist, wobei in die Aufnahmebohrung ein mit einer Druckluftquelle verbundener Luftzufuhrkanal und ein mit einer Flüssigkeitsversorgung verbundener Spülflüssigkeitszufuhrkanal münden sowie von dieser ein Luftaustrittskanal und ein Spülflüssigkeitsaustrittskanal ausgehen, die zu einem distalen Ende des Endoskops führen, wobei der Ventilschieber mit einem proximalen, axial über einen den Ventilschieber umschließenden Haltering vorstehenden Betätigungsabschnitt versehen ist, welcher an seinem Ende mit einem Betätigungsknopf verbunden ist, wobei der Betätigungsknopf einerseits am Haltering über eine Druckfeder abgestützt ist und andererseits mit dem Haltering ein ringförmiger Vorsprung des Ventilschiebers als in axialer Richtung wirkender Anschlag zusammenwirkt, und wobei am distalen Steuerungsabschnitt des Ventilschiebers Dichtringe vorgesehen sind, die radial über den Ventilschieber vorstehen und gleitend an einer Innenmantelfläche der Aufnahmebohrung geführt sind, um Steuerräume der Ventileinheit zueinander abzudichten.

Schließlich betrifft die Erfindung auch ein Verfahren zur Herstellung eines Ventilschiebers für eine aus einem Insufflations- und einem Spülventil bestehende Ventileinheit eines medizinischen Endoskops, der einen proximalen Betätigungsabschnitt aufweist, wobei an einem distalen Steuerungsabschnitt des Ventilschiebers Dichtringe vorgesehen sind.

### Stand der Technik

In der flexiblen Endoskopie verwendete Endoskope werden an ihrem proximalen Handbetätigungsteil mit einer aus einem Insufflations- und einem Spülventil bestehenden Ventileinheit versehen, die in der Regel aus einem verschiebbar in einer Aufnahmebohrung des Handbetätigungsteils angeordneten Ventilschieber besteht. Alternativ dazu kann die Ventileinheit auch ein Ventilgehäuse aufweisen, mit dem sie in die Aufnahmebohrung des Handbetätigungsteils eingesetzt ist.

Bei der Insufflationsfunktion wird der Eintritt eines gasförmigen Mediums, beispielsweise Luft oder CO₂ in einen Hohlraum im Körper des Patienten, in dem ein endoskopischer Eingriff vorgenommen werden soll, gesteuert, wobei das in diesen einströmende gasförmige Medium dafür sorgt, dass sich dessen Volumen vergrößert. Das gasförmige Medium wird der Ventileinheit aus einer Gasflasche oder einem Druckluftsystem zugeführt. Ein mittels des Ventilschiebers gesteuerter Austrittskanal für das gasförmige Medium ist bis an ein distales Ende des Endoskops geführt. Wenn als gasförmiges Druckmittel Luft verwendet wird, ist der Ventilschieber zumeist derart ausgebildet, dass die Luft dann, wenn kein Insufflationsvorgang durchgeführt wird, über eine Längsbohrung, die an einem Betätigungsknopf aus diesem austritt.

Weiterhin weist der Ventilschieber einen Abschnitt auf, der Bestandteil eines Spülventils ist. Dem Spülventil wird eine mit einem Druck beaufschlagte Spülflüssigkeit aus einem Vorratsbehälter zugeführt, wobei die Spülflüssigkeit in dessen geöffneter Stellung über einen Spülflüssigkeitsaustrittskanal ebenfalls an das distale Ende des Endoskops geleitet wird. Die aus dem Endoskop austretende Spülflüssigkeit gelangt dabei an eine Oberfläche eines am distalen Ende des Endoskops vorgesehenen Fensters, hinter welchem sich eine Optik des Endoskops befindet und reinigt dieses.

Ein Ventilschieber für eine aus einem Insufflations- und einem Spülventil bestehende Ventileinheit, eine Ventileinheit mit einem entsprechenden Ventilschieber sowie ein Verfahren zur Herstellung eines Ventilschiebers der jeweils im Oberbegriff der Patentansprüche 1, 2 und 10 angegebenen Gattung sind aus der WO 2012/075131 A1 bekannt. Dabei weist der als Hauptschaft bezeichnete Ventilschieber, der längsverschieblich in einer Aufnahmebohrung angeordnet sein soll, eine Vielzahl von an diesem in Umfangsrichtung verlaufenden Rippen und Nuten auf, die monolithisch mit dem Hauptschaft ausgebildet sind. In den Nuten sind Dichtungen angeordnet, die dadurch hergestellt und in den Nuten angebracht werden sollen, dass der mit der jeweiligen Nut versehene Bereich des Ventilschiebers mit dem Dichtungswerkstoff umspritzt wird.

Dadurch wird erreicht, dass die Dichtung von der Nut aufgenommen ist, während ein radial über die Nut vorstehender Abschnitt der Dichtung gleitend an einer Innenmantelfläche der Aufnahmebohrung geführt ist und innerhalb der Ventileinheit Druckräume zueinander abdichtet. Ein proximaler Teilbereich des Ventilschiebers ist mit einer zentrisch verlaufenden Längsbohrung versehen, von der distal eine Querbohrung ausgeht und die am proximalen Ende des Ventilschiebers austritt. Über die gesamte Länge ist der Ventilschieber mit einer Vielzahl von Durchmesserstufen versehen, um die Nuten und Rippen sowie kolbenartige Vorsprünge zu bilden. Mit einem abgesetzten proximalen Endabschnitt ist der Ventilschieber in eine Muffe eingesetzt, wobei der Endabschnitt eine im Inneren der Muffe vorgesehene Membran durchstößt und an seinem stirnseitigen Ende mit einem ebenfalls eine zentrische Bohrung aufweisenden Betätigungsknopf versehen ist.

Die Insufflationsfunktion der Ventileinheit wird dadurch gesteuert, dass die zentrische Bohrung und somit die im Ventilschieber verlaufende Längsbohrung durch einen Finger der das Endoskop bedienenden Person verschlossen werden. Dadurch strömt das gasförmige Medium von einem Luftzufuhrkanal in einen Luftaustrittskanal der Ventileinheit. Wird der Finger von der Bohrung abgehoben, tritt das gasförmige Medium über diese aus der Ventileinheit aus. An seinem distalen Endabschnitt bildet der Ventilschieber mit der Aufnahmebohrung einen Steuerraum, über den ein Spülflüssigkeitszufuhrkanal und ein Spülflüssigkeitsaustrittskanal miteinander verbindbar sind.

Weiterhin sind aus der US 4,748,970 A und der WO 2017091459 A9 jeweils eine aus einem Insufflations- und einem Spülventil bestehende Ventileinheit bekannt, die hinsichtlich ihres Aufbaus und ihrer Funktion im Wesentlichen mit der Ventileinheit nach der WO 2012/075131 A1 übereinstimmt. Dabei sind Nuten und Rippen ebenfalls monolithisch mit einem Hauptschaft bzw. Ventilschieber ausgebildet. Nach der US 4,748,970 A dient eine Dichtung, die in eine Nut des Hauptschafts eingesetzt ist, als Rückschlagventil zwischen einem Luftaustrittskanal und einem Lufteintrittskanal. Im Übrigen soll offenbar auf Dichtungen verzichtet werden, indem der Ventilschieber dichtend in einem Zylinder geführt ist. Nach der WO 2017091459 A9 sind mehrere Dichtungen und Führungsringe in Nuten des Ventilschiebers eingesetzt. Unter anderem ist für die Steuerung des Spülflüssigkeitsstromes eine Dichtung mit zwei Dichtlippen vorgesehen, die über einen zylindrischen Abschnitt miteinander verbunden sind.

### Offenbarung der Erfindung

Es ist Aufgabe der vorliegenden Erfindung, einen Ventilschieber für eine aus einem Insufflations- und einem Spülventil bestehende Ventileinheit zu schaffen, der kostengünstig in Großserie im Spritzgussverfahren herstellbar ist und hinsichtlich seiner Funktionalität Vorteile aufweist.

Diese Aufgabe wird, ausgehend vom jeweiligen Oberbegriff der Patentansprüche 1, 2 und 10 in Verbindung mit dessen kennzeichnenden Merkmalen gelöst. Die von den Patentansprüchen 1 und 2 abhängigen Patentansprüche beinhalten erfindungsgemäße Weiterbildungen dieser Lösungen.

Danach soll ein Ventilschieber für eine als Insufflations- und Spülventil dienende Ventileinheit eines medizinischen Endoskops einen distalen Steuerungsabschnitt aufweisen und in eine mit Luftzufuhr- und Luftaustrittskanälen sowie Spülflüssigkeitszufuhr- und Spülflüssigkeitsaustrittskanälen verbundene, zumindest mittelbar in einem Handbetätigungsteil eines Endoskops ausgebildete Aufnahmebohrung einsetzbar sein. Der Ventilschieber weist einen proximalen Betätigungsabschnitt auf, der durch einen Haltering hindurchgeführt sowie mit einem Betätigungsknopf versehen ist, wobei einerseits am Haltering der Betätigungsknopf über eine Druckfeder abgestützt ist und andererseits mit diesem ein ringförmiger Vorsprung des Ventilschiebers als in axialer Richtung wirkender Anschlag zusammenwirkt.

Am distalen Steuerungsabschnitt des Ventilschiebers, der axial über den Haltering vorsteht, sind Dichtringe vorgesehen, die radial über den Ventilschieber vorstehen und vorgesehen sind, um, gleitend an einer Innenmantelfläche der Aufnahmebohrung geführt, Steuerräume der Ventileinheit zueinander abzudichten. Folglich dienen die vorzugsweise mit Dichtlippen versehenen Dichtringe dazu, die Steuerräume, denen Druckmittel zugeführt wird, abzudichten, damit über den Ventilschieber die bis an das distale Ende des Endoskops geführten Ströme des für einen Insufflationsvorgang erforderlichen gasförmigen Mediums und des für einen Spülvorgang erforderlichen Flüssigkeitsstrom steuerbar sind.

Erfindungsgemäß soll dabei eine Außenmantelfläche des distalen Steuerungsabschnittes, abgesehen von an dieser eventuell vorgesehenen radialen Führungsabschnitten, glattwandig ausgebildet sein, und die Dichtringe sollen stoffschlüssig mit der glattwandigen Außenmantelfläche verbunden sein. An der Außenmantelfläche sind somit keine Mittel vorgesehen, mittels welcher ein Formschluss zwischen dem Ventilschieber und den Dichtringen erzielt wird. Diese sind ausschließlich über einen Stoffschluss an der Außenmantelfläche fixiert.

Daraus ergeben sich erhebliche Vorteile bei der Herstellung des Ventilschiebers. Die glattwandige Oberfläche ist einzig und allein durch Führungsabschnitte unterbrochen, die radial über die Außenmantelfläche vorstehen und dazu dienen, den Ventilschieber in einer Aufnahmebohrung zu führen. Durch den radialen Abstand zwischen der Außenmantelfläche des Ventilschiebers und einer Innenmantelfläche der Aufnahmebohrung werden die Steuerräume geschaffen. Es besteht natürlich auch die Möglichkeit, die Führungsabschnitte an der Innenmantelfläche vorzusehen, wobei dann bei dem glattwandigen distalen Steuerungsabschnitt des Ventilschiebers auch auf einen radialen Bund oder Vorsprünge verzichtet werden kann.

Weiterhin soll eine Ventileinheit eines medizinischen Endoskops, die als Insufflationsventil und Spülventil mit einem gemeinsamen Ventilschieber dient, mit einem distalen Steuerungsabschnitt verschiebbar in einer Aufnahmebohrung eines Gehäuses angeordnet sein. Dabei münden in die Aufnahmebohrung ein mit einer Druckluftquelle verbundener Luftzufuhrkanal und ein mit einer Flüssigkeitsversorgung verbundener Spülflüssigkeitszufuhrkanal sowie gehen von dieser ein Luftaustrittskanal und ein Spülflüssigkeitsaustrittskanal aus, die zu einem distalen Ende des Endoskops führen. Der Ventilschieber ist mit Steuerabschnitten und einem proximalen, axial über einen den Ventilschieber umschließenden Haltering vorstehenden Betätigungsabschnitt versehen, welcher an seinem Ende mit einem Betätigungsknopf verbunden ist.

Dieser Betätigungsknopf ist einerseits am Haltering über eine Druckfeder abgestützt, und es wirkt andererseits mit diesem ein ringförmiger Vorsprung des Ventilschiebers als in axialer Richtung wirkender Anschlag zusammen. Am distalen Steuerungsabschnitt des Ventilschiebers sind Dichtringe vorgesehen, die radial über den Ventilschieber vorstehen und gleitend an einer Innenmantelfläche der Aufnahmebohrung geführt sind, um Steuerräume der Ventileinheit zueinander abzudichten. Als Steuerabschnitte sind Bereiche bezeichnet, die in axialer Richtung durch die Dichtringe begrenzt sind, so dass gemeinsam mit der Aufnahmebohrung Steuerräume begrenzt sind.

Dabei soll ebenfalls eine Außenmantelfläche des distalen Steuerungsabschnittes, abgesehen von an dieser eventuell vorgesehenen radial vorstehenden Führungsabschnitten, glattwandig ausgebildet sein, wobei die Dichtmittel stoffschlüssig mit der glattwandigen Außenmantelfläche verbunden sind. In diesem Fall ist ein erfindungsgemäß ausgebildeter Ventilschieber Bestandteil einer Ventileinheit.

Demgegenüber sind nach der WO 2012/075131 A1 sowie den weiteren Druckschriften US 4,748,970 A und der WO 2017091459 A9 die Dichtringe jeweils in Nuten des für den Ventilschieber vorgesehenen Hauptkörpers angeordnet, damit diese durch einen Formschluss in ihrer Position am Hauptkörper gehalten werden. Da der jeweilige Hauptkörper einen zentrisch verlaufenden Längskanal aufweist, ist für eine Ausbildung derartiger Nuten eine relativ große Wandstärke zwischen dem Längskanal und dessen Außenmantelfläche vorzusehen, was bei den kleinen Abmessungen der Ventileinheit schwierig ist. Außerdem müssen die Ventileinheiten vor einer Verwendung in einem Endoskop sterilisiert werden, damit diese frei von Keimen ist. Im Vergleich zu einer glatten Oberfläche, mit der Dichtringe stoffschlüssig verbunden sind, ist es deutlich schwieriger, einen fertigen Ventilschieber, der Dichtringe formschlüssig aufnehmende nuten aufweist, zu sterilisieren.

Außerdem soll erfindungsgemäß der Ventilschieber als prismenartiger Hohlkörper mit einem kreisringförmigen Querschnitt ausgebildet sein, in dem zur Leitung der Druckluft ein Längskanal vorgesehen ist. Der Hohlkörper soll im Spritzguss aus einem thermoplastischen Kunststoff hergestellt sein. Da in dem Hohlkörper keine Nuten vorgesehen sind, kann dieser insgesamt eine geringe und nahezu konstante Wandstärke aufweisen.

In weiterer Ausgestaltung der Erfindung ist vorgesehen, dass der Hohlkörper aus Acryl Butadien Styrol und die Dichtringe aus einem thermoplastischen Elastomer hergestellt sind. Somit werden die beiden Komponenten aus Werkstoffen hergestellt, deren Schmelzpunkte nahe beieinander liegen. Daher kann beim Anspritzen der Dichtungen, bei dem der Hohlkörper eine Oberflächentemperatur von 60 bis 100°C aufweist, ein optimaler Stoffschluss erzielt werden.

Weiterhin ist vorgesehen dass der Hohlkörper zumindest im distalen Bereich des Steuerungsabschnittes des Ventilschiebers kegelstumpfförmig ausgebildet ist. An diesen ersten kegelstumpfförmigen Teilabschnitt kann sich in axialer Richtung ein weiterer kegelstumpfförmiger Teilabschnitt anschließen. Die radialen Abmessungen der stufenartig ausgebildeten Aufnahmebohrung sind an diese radialen Abmessungen des Ventilschiebers angepasst, d.h., ein distal letzter Steuerungsabschnitt weist den geringsten Durchmesser auf.

Außerdem sollen die Führungsabschnitte als am Umfang des Hohlkörpers gleichmäßig verteilte Kufen ausgeführt sind, die durch sich vom Umfang aus radial erstreckende Ansätze gebildet werden. Dadurch ist der Ventilschieber aufgrund der reduzierten Führungsfläche mit geringer Reibung in der Aufnahmebohrung verschiebbar.

In einem zwischen zwei Dichtringen liegenden Bereich soll in der Wandung des Hohlkörpers zumindest eine mit dem Längskanal kommunizierende Querbohrung vorgesehen sein. Über diese Queröffnung wird die Druckluft der Längsbohrung zugeführt. Der Luftzufuhrkanal ist dabei permanent mit einem stirnseitig des Betätigungsabschnitts mündenden Steuerkanal verbunden ist. Dieser Steuerkanal liegt in einer Stirnseite eines am Ventilschieber fixierten Betätigungsknopfes und kann zur Steuerung des Druckluftstromes für eine Insufflation mit dem Finger abgedeckt werden.

Außerdem ist vorgesehen, dass ein zur Steuerung des Druckluftstromes dienender Dichtring, der in beiden Schaltstellungen des Ventilschiebers eine Lage zwischen dem Luftzufuhrkanal und dem Luftaustrittskanal einnimmt, als Rückschlagventil wirkt und nur einen Druckluftstrom vom Luftzufuhrkanal zum Luftaustrittskanal freigibt. Dabei kann es sich um einen Nutring handeln, dessen Dichtlippe druckabhängig von dem in der Aufnahmebohrung vorgesehenen Dichtbereich abhebt.

Die den Ventilschieber aufnehmende Aufnahmebohrung soll in einem ihrem distalen Ende benachbarten axialen Abschnitt, der an den Spülflüssigkeitszulaufkanal angeschlossen ist, gegenüber einem sich daran in proximaler Richtung anschließenden Dichtbereich radial erweitert sein. Bei einer manuellen axialen Verschiebung des Ventilschiebers gelangt der distal letzte Dichtring in diesen radial erweiterten Bereich der Aufnahmebohrung, so dass Spülflüssigkeitszulaufkanal und Spülflüssigkeitsaustrittskanal für einen Spülvorgang miteinander verbunden sind.

Weiterhin wird die Aufgabe auch bei einem Verfahren zur Herstellung eines Ventilschiebers für eine aus einem Insufflations- und einem Spülventil bestehende Ventileinheit eines medizinischen Endoskops, der kolbenschieberartig ausgebildet ist und einen proximalen Betätigungsabschnitt aufweist, gelöst, wobei an einem distalen Steuerungsabschnitt des Ventilschiebers Dichtringe vorgesehen sein sollen.

Dabei ist vorgesehen, dass der als prismenartiger Hohlkörper mit kreisringförmigem Querschnitt ausgebildete Ventilschieber in einem ersten Arbeitsschritt in einer ersten, einen Kern aufnehmenden Spritzgießform mit einer glattwandig ausgebildeten Außenmantelfläche, an der radiale Führungsabschnitten vorgesehen sind, herstellt wird, und dass dieser prismenartige Hohlkörper im noch nicht erkalteten Zustand in einem zweiten Arbeitsschritt in eine zweite, Außenkonturen der Dichtringe aufweisende Spritzgießform eingesetzt wird und die Dichtringe an die glattwandige Außenmantelfläche des Hohlkörpers angespritzt werden. Die Spritzgießformen bestehen vorzugsweise aus zwei Formhälften mit jeweils zwei Kavitäten, so dass jeder der Verfahrensschritte zur Herstellung von zwei Ventilschiebern dient.

In weiterer Ausgestaltung dieses Verfahrens sollen die Schmelzpunkte der für den Hohlkörper und die Dichtringe verwendeten Werkstoffe nahe beieinander liegen. Der Schmelzpunkt ist die Temperatur, bei der ein Werkstoff von seinem festen in den flüssigen Aggregatzustand übergeht. Demzufolge können als Werkstoff des Hohlkörpers Acryl Butadien Styrol und der Dichtringe ein thermoplastisches Elastomer vorgesehen sein. Der Hohlkörper soll beim Anspritzen der Dichtringe eine Oberflächentemperatur von 60 - 100° aufweisen.

Die Erfindung ist nicht auf die angegebene Kombination der Merkmale der unabhängigen Patentansprüche 1, 2 und 12 mit den auf diese rückbezogenen Patentansprüchen beschränkt. Es ergeben sich darüber hinaus Möglichkeiten, einzelne Merkmale, auch soweit sie aus den Patentansprüchen, den Vorteilsangaben zu den Patentansprüchen, der nachfolgenden Beschreibung der Ausführungsbeispiele oder zumindest aus der Zeichnung hervorgehen, miteinander zu kombinieren. Die Bezugnahme der Patentansprüche auf die Zeichnung durch die Verwendung von Bezugszeichen soll den Schutzumfang der Patentansprüche nicht beschränken.

### Kurze Beschreibung der Zeichnung

Zur weiteren Erläuterung der Erfindung wird auf die Figuren verwiesen, in denen ein Ausführungsbeispiel der Erfindung vereinfacht dargestellt ist. Es zeigen:
- Figur 1: eine schematische Teilansicht eines Endoskops mit einer in diesem angeordneten Ventileinheit, die im Längsschnitt dargestellt ist,
- Figur 2: eine Seitenansicht eines Ventilschiebers der Ventileinheit, der in einem Haltering angeordnet ist,
- Figur 3: einen Längsschnitt gemäß Linie III - III durch den Ventilschieber und den Haltering in Figur 2,
- Figur 4: eine Seitenansicht eines für die Herstellung des Ventilschiebers vorgesehenen Hohlkörpers,
- Figur 5: einen Längsschnitt gemäß Linie V - V durch den Hohlkörper in Figur 4,
- Figur 6: einen Querschnitt gemäß Linie VI - VI durch den Hohlkörper in Figur 4,
- Figur 7: einen Querschnitt gemäß Linie VII - VII durch den Hohlkörper in Figur 4,
- Figur 8: die im Längsschnitt dargestellte Ventileinheit in einem ersten Schaltzustand, in welchem einem distalen Ende des Endoskops weder ein gasförmiges Medium noch eine Spülflüssigkeit zugeführt werden,
- Figur 9: im Längsschnitt die Ventileinheit, wobei diese stirnseitig mit einem Finger abgedeckt wird, so dass ein Austritt des gasförmigen Mediums über einen Betätigungsknopf verhindert ist und dieses folglich in das Endoskop strömt,
- Figur 10: im Längsschnitt die Ventileinheit, wobei der Ventilschieber derart in Längsrichtung verschoben ist, dass die Spülflüssigkeit aus einem Spülflüssigkeitszufuhrkanal über den Ventilschieber einem Spülflüssigkeitsaustrittskanal und somit dem Endoskop zugeführt wird,
- Figur 11: eine Draufsicht auf eine untere Hälfte einer Spritzgießform zur Herstellung des Hohlkörpers und
- Figur 12: eine Draufsicht auf eine untere Hälfte einer Spritzgießform mit in dieser angeordnetem Hohlkörper, wobei in dieser Spritzgießform Dichtringe an den Hohlkörper angespritzt werden.

### Ausführliche Beschreibung der Zeichnung

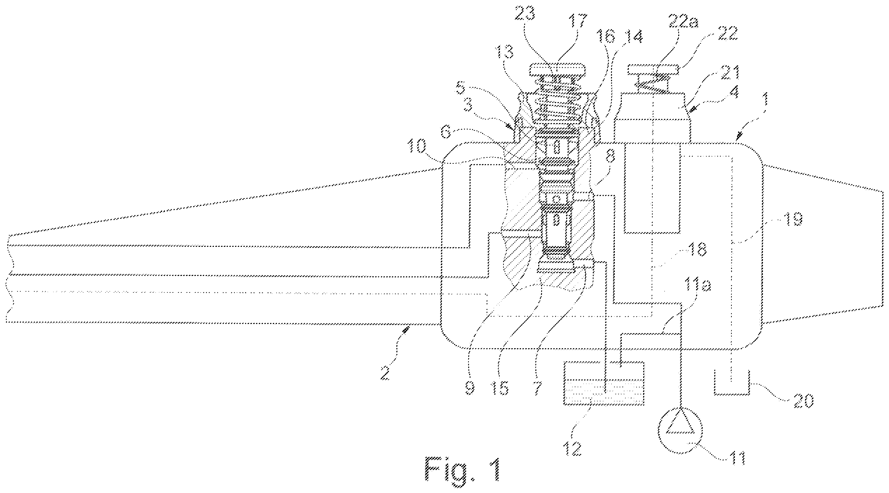

In der Figur 1 ist mit 1 ein Handbetätigungsteil eines Endoskops 2 bezeichnet, in dem eine erfindungsgemäß ausgebildete Ventileinheit 3, welche als Insufflationsventil und Spülventil dient, angeordnet ist. Das Endoskop 2 weist einen nicht näher dargestellten Einführschlauch auf, dessen distales Ende in einen Gastrointestinaltrakt eines zu behandelnden Patienten einführbar ist. Weiterhin nimmt das proximal am Endoskop 2 angeordnete Handbetätigungsteil 1 ein Entleerventil 4 auf.

Die Ventileinheit 3 weist einen Ventilschieber 5 auf, der längsverschiebbar in einer Aufnahmebohrung 6 des Handbetätigungsteils 1 angeordnet ist. Dabei münden in die Aufnahmebohrung 6 ein Spülflüssigkeitszufuhrkanal 7 und ein Luftzufuhrkanal 8, während von der Aufnahmebohrung 6 ein Spülflüssigkeitsaustrittskanal 9 und ein Luftaustrittskanal 10 ausgehen, die bis an das nicht näher dargestellte distale Ende des Endoskops 2 verlaufen. An Stelle von Luft kann auch ein anderes gasförmiges Medium, beispielsweise CO₂ für die Insufflation verwendet werden.

Der Spülflüssigkeitszufuhrkanal 7 und der Luftzufuhrkanal 8 sind vorzugsweise Bestandteil einer vom Handbetätigungsteil 1 ausgehenden Versorgungsleitung. Mittels dieser ist das Endoskop 2 mit einer Druckluftquelle 11 verbunden, die zum einen Druckluft in den Luftzufuhrkanal 8 fördert und zum anderen über eine Druckleitung 11a einen Spülflüssigkeitsvorratsbehälter 12 mit Druck beaufschlagt, so dass die in diesem befindliche Spülflüssigkeit in den Spülflüssigkeitszufuhrkanal 7 verdrängt wird.

Spülflüssigkeit, die in einer geöffneten Stellung eines als Spülventil dienenden Abschnitts der Ventileinheit 3 in den Spülflüssigkeitsaustrittskanal 9 gelangt, wird am distalen Ende des Endoskops an eine Oberfläche eines nicht näher dargestellten Fensters geleitet, hinter welchem sich eine Optik des Endoskops 1 befindet, so dass dieses Fenster gereinigt wird. Die in den Luftaustrittskanal 10 gelangende Luft, bei der es sich alternativ auch um ein anderes gasförmiges Medium, wie zum Beispiel CO₂ handeln kann, wird über das distale Ende des Endoskops 2 in einen Hohlraum im Körper des Patienten geleitet, um dessen Volumen im Rahmen einer Insufflation zu vergrößern.

Der Ventilschieber 5 ist mit einem proximalen Endabschnitt in einem Halteringes 13 geführt, wobei dieser an einem axialen Teilabschnitt von einer elastischen Muffe 14 umschlossen ist. Ein Gehäuse 15 des Handbetätigungsteils 1 weist einen vorstehenden Bund 16 auf, der dabei von der Muffel 14 übergriffen wird. Wie weiterhin der Darstellung entnommen werden kann, nimmt der Ventilschieber 5 an seinem proximalen Ende einen Betätigungsknopf 17 auf. Dieser stützt sich und somit den Ventilschieber 5 über eine Druckfeder 23 an dem Haltering 13 ab. Die weiteren Einzelheiten der Ausgestaltung der Ventileinheit 3 sollen nachfolgend anhand der weiteren Figuren erläutert werden.

Das bereits genannte Entleerventil 4 ist als 2/2-Wegeventil ausgebildet und steht über einen Entleerkanal 18 mit dem distalen Ende des Endoskops 2 in Verbindung, wobei es in seinem geöffneten Zustand Flüssigkeit aus dem Hohlraum des Körpers über einen Sammelkanal 19 in ein Sammelreservoir 20 leitet. Dabei weist das Entleerventil 4 ebenfalls einen in einem Halteringes 21 verschiebbaren Ventilschieber auf, der mittels eines Druckknopfes 22 betätigbar und über eine Druckfeder 22a in axialer Richtung am Halteringes 21 abgestützt ist.

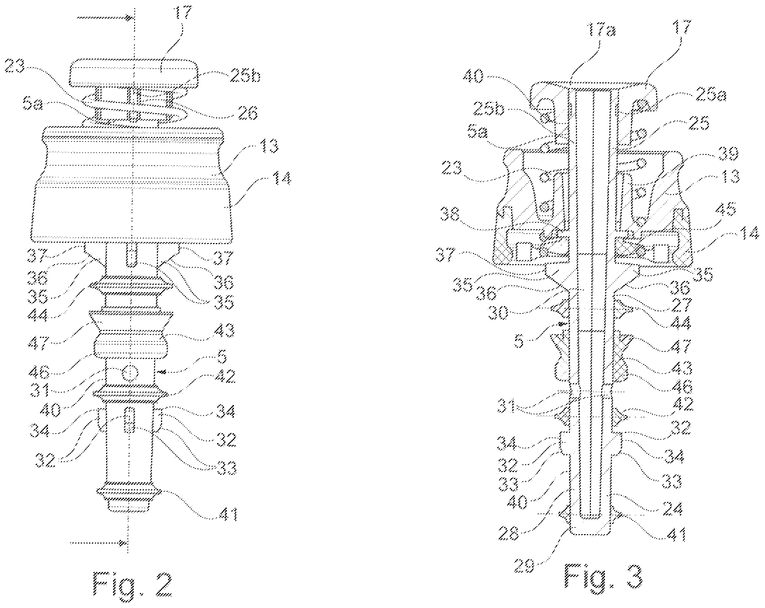

In den Figuren 2 und 3 ist die aus dem Ventilschieber 5 und dem Halteringes 13 bestehende Einheit, die Bestandteil der in Figur 1 dargestellten Ventileinheit 5 ist, dargestellt. Dabei geht insbesondere aus der Figur 3 hervor, dass der Ventilschieber 5 aus einem prismenartigen Hohlkörper 24 mit kreisringförmigem Querschnitt besteht, wobei sich der Hohlkörper 24, in Längsrichtung gesehen, aus drei unterschiedlich ausgebildeten Teilabschnitten zusammensetzt. In einem proximalen Teilabschnitt ist der Hohlkörpers 24 im Wesentlichen zylindrisch ausgebildet, d.h., dessen Außenwand 25 verläuft, abgesehen von einer endseitigen Nut 25a und längsverlaufenden Rippen 25b, mit konstantem Innen- bzw. Außendurchmesser. Eine Außenmantelfläche 40 des Hohlkörpers 24 ist glattwandig ausgebildet.

Daran schließt sich ein mittlerer Bereich des Hohlkörpers 24 an, in welchem ein zweiter als umlaufende Außenwand 27, in Längsrichtung gesehen, unter einen geringen Winkel von der Zylinderform abweicht, also kegelstumpfförmig ausgeführt ist. Schließlich schließt sich an diesen zweiten Bereich ein dritter Bereich an, in dem eine dritte Außenwand 28 unter einer stärkeren Neigung als die Außenwand 27, ebenfalls kegelstumpfförmig verläuft. Die axialen Erstreckungen der unterschiedlich ausgebildeten Abschnitte mit den entsprechend verlaufenden Außenwänden 25, 27 und 28 sind durch quer innerhalb eines Längskanals 30 verlaufende Linien gekennzeichnet.

An seinem distalen Ende ist der prismenartige Hohlkörper 24 durch eine stirnseitige Wand 29 verschlossen. Insgesamt wird der Längskanal 30 durch die vorstehend beschriebene hohle Ausbildung des den Ventilschieber 5 bildenden Hohlkörpers 24 geschaffen, der sich innerhalb des Hohlkörpers 24 axial von der Wand 29 bis an dessen proximales Ende erstreckt. Von diesem Längskanal 30 gehen zwei Querbohrungen 31 aus, die in der Außenwand 28 des dritten Teilabschnitts ausgebildet sind. Weiterhin ist diese Außenwand 28 mit ersten Kufen 32 versehen, die sich jeweils radial von der Außenwand 28 aus erstrecken und um jeweils 90° zueinander umfangsversetzt angeordnet sind. Jede Kufe 32 weist eine Anfasung 33 und im Übrigen ein konzentrisch zur Außenwand 28 verlaufendes Ende 34 auf.

An der Außenwand 27 des mittleren Teilabschnitts des Hohlkörpers 24 sind zweite Kufen 35 vorgesehen, die sich von diesem aus ebenfalls radial erstrecken und um 90° zueinander umfangsversetzt sind. Auch diese zweiten Kufen 35 weisen jeweils eine Anfasung 36 und ein konzentrisch zur Außenwand 27 verlaufendes Ende 37 auf. Die über die Enden der diametral verlaufenden Kufen 32 sowie 35 gemessene Spannweite ist bei den zweiten Kufen 35 größer als bei den ersten Kufen 32. Im Übrigen ist an der Außenwand 25 des ersten Teilabschnitts ein radial vorstehender, ringförmiger Vorsprung 38 ausgebildet, der wie außerdem aus der Figur 3 hervorgeht, als axialer Anschlag zwischen dem Ventilschieber 5 und dem Halteringes 13 wirkt.

Der Ventilschieber 5 ist mit seinem proximalen Ende 5a durch eine Nabe 39 des Halterings 13 hindurchgeführt, wobei sich die zum Teil innerhalb des Halterings 13 verlaufende, den Hohlkörper 24 an einem axialen Abschnitt umschließende Druckfeder 23 einerseits am Halteringes 13 und andererseits am Betätigungsknopf 17 abstützt. Der Betätigungsknopf 17 ist dabei auf einer Außenmantelfläche 40 des Hohlkörpers 24 fixiert, wobei Längsschlitze 26 des Betätigungsknopfes 17 die Rippen 25b umgreifen und der Längskanal 30 an einer muldenartig ausgebildete Stirnseite 17a dieses Betätigungsknopfes 17 mündet.

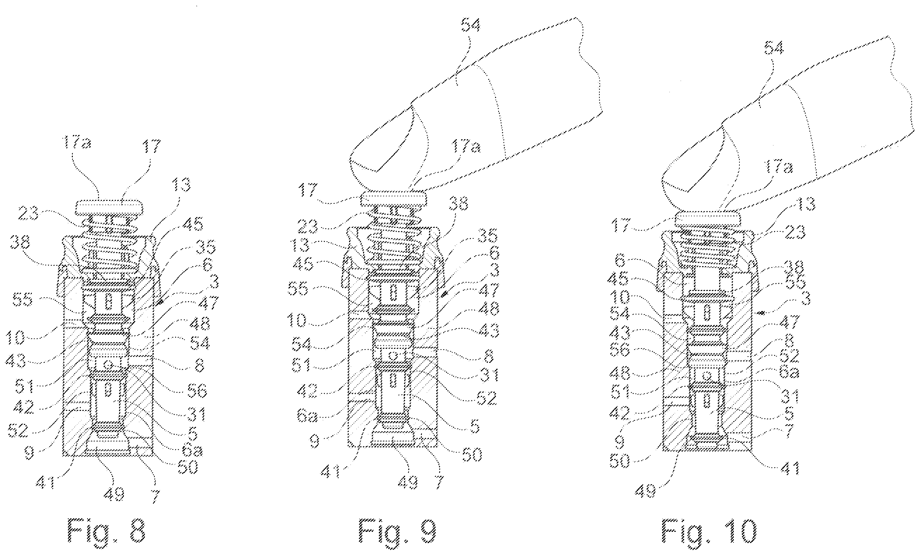

Wesentlich ist eine glattwandige Ausbildung der Außenmantelfläche 40 und eine stoffschlüssige Verbindung der Dichtringe 41, 42, 43 44 und 45 mit dieser. Dabei weist der Dichtring 43 einen distalen Teilbereich 46 auf, in dem dieser als Sitzdichtring wirkt, wie insbesondere die nachfolgende Figur 10 zeigt. Ein proximaler Teilbereich 47 des Dichtrings 43 ist als Nutring ausgebildet und wirkt, wie die nachfolgenden Figuren 9 und 10 zeigen, als Rückschlagventil für die durch die Ventileinheit 3 strömende Luft. Die übrigen Dichtringe 41, 42, 44 und 45 weisen jeweils zumindest eine radiale Dichtlippe auf.

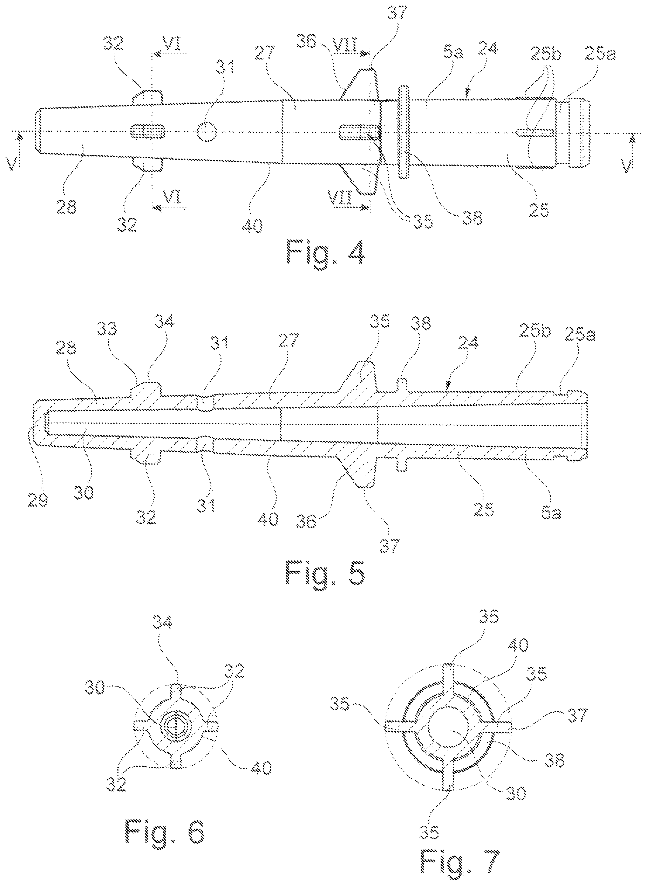

Die Figuren 4, 5 ,6 und 7 zeigen den prismenartigen Hohlkörper 24 sowie Schnittdarstellungen von diesem. Der Hohlkörper 24 ist in den Figuren 4 und 5 in einem Zustand dargestellt, bevor die Dichtringe 41, 42,43, 44 und 45 stoffschlüssig mit dessen Außenmantelfläche 40 verbunden werden. Zunächst zeigt die Figur 4 eine Seitenansicht des prismenartigen Hohlkörpers 24 mit den ersten Kufen 32, den zweiten Kufen 35 und dem ringförmigen Vorsprung 38.

Der Darstellung kann entnommen werden, dass die Außenmantelfläche 40 des Hohlkörpers 24 in den drei unterschiedlichen Teilbereichen der Längserstreckung unterschiedlich verläuft. Im ersten Teilbereich, in dem der Hohlkörper 24, wie die Figur 5 zeigt, durch die erste Außenwand 25 gebildet wird, ist dieser im Wesentlichen zylindrisch gestaltet. Im zweiten und dritten Teilbereich des Hohlkörpers 24, in dem dieser die Außenwände 27 und 28 aufweist, verlaufen diese dagegen mit unterschiedlicher Neigung kegelstumpfförmig. Die Figuren 6 und 7 verdeutlichen in entsprechenden Querschnitten durch den Hohlkörper 24 die Ausbildung der Kufen 32 und 35.

In den weiteren Figuren 8 bis 10 ist die Ventileinheit 3 im Gegensatz zur Ausführung nach der Figur 1 mit einem Ventilgehäuse 48 versehen, so dass die gesamte Ventileinheit in das entsprechend gestaltete Handbetätigungsteil 1 einsetzbar ist. Im Ventilgehäuse 48 verläuft die Aufnahmebohrung 6, welche den längsverschiebbaren Ventilschieber 5 aufnimmt. Die Aufnahmebohrung 6 ist insgesamt stufenartig ausgebildet und an ihrem distalen Ende zur Bildung eines an den Spülflüssigkeitszulaufkanal 8 angeschlossenen Zulaufraumes 49 radial erweitert. Daran schließt sich ein an einer Innenmantelfläche 6a der Aufnahmebohrung 6 ausgebildeter Dichtbereich 50 an, mit dem, je nach Stellung des Ventilschiebers 5, der Dichtring 41 zusammenwirken kann.

Ein Steuerraum 51 ist zwischen den Dichtringen 42 und 43 ausgebildet, wobei, wie bereits im Zusammenhang mit der Figur 3 dargelegt, der Dichtring 43 die Funktion eines Rückschlagventils übernehmen kann. Der als Nutring ausgebildete Teilbereich 47 des Dichtringes 43 wirkt dabei mit einem an der Innenmantelfläche 6a gebildeten Dichtbereich 52 zusammen, während der als Sitzdichtring ausgebildete Teilbereich seine Dichtfunktion gemeinsam mit einem am Ende des Dichtbereichs 52 vorgesehenen Ventilsitzes 56 erfüllt. Oberhalb des vorgenannten Dichtbereichs 52 befindet sich ein durch eine nochmalige radiale Erweiterung der Aufnahmebohrung 6 geschaffener Dichtbereich 55, in dem die Kufen 35 geführt sind und mit dem zur Abdichtung des Ventilgehäuses 48 in proximaler Richtung der Dichtring 45 zusammenwirkt.

In der Figur 8 befindet sich die Ventileinheit 3 zunächst in ihrem unbetätigten Zustand, in dem der ringförmige Vorsprung 38 unter der Vorspannung der Druckfeder 23 am Haltering 21 anliegt. In dieser Stellung des Ventilschiebers 5 gelangt die unter Druck aus dem Luftzufuhrkanal 8 in den Steuerraum 51 eintretende Luft aus diesem über die Querbohrung 31 in den Längskanal (Längskanal 30 in Figur 3). Daher tritt die der Ventileinheit 3 zugeführte Luft drucklos aus dem proximalen Ende des Ventilschiebers 5, nämlich in der Mitte des Betätigungsknopfes 17, aus. Der Dichtring 41 liegt dabei dichtend im Dichtbereich 50 der Aufnahmebohrung 6 an, so dass der Spülflüssigkeitsaustrittskanal 9 gegenüber dem Spülflüssigkeitszufuhrkanal 7 abgesperrt ist. Es gelangt somit weder Druckluft für eine Insufflation noch Spülflüssigkeit für einen Spülvorgang an das distale Ende des Endoskops 2.

Wird nun, wie in Figur 9 dargestellt, von der das Endoskop bedienenden Person eine Fingerkuppe eines Fingers 54 auf die Stirnseite 17a des Betätigungsknopfes 17 gelegt, so führt das dazu, dass die Druckluft nicht mehr über den zuvor beschriebenen Längskanal 30 austreten kann. Damit wird erreicht, dass sich im Steuerraum 51 ein Druck aufbaut, der bewirkt, dass der Teilbereich 47 des Dichtrings 43, der als Nutring ausgebildet ist, von einem mit diesem zusammenwirkenden Dichtbereich 54 abhebt. Somit kann über den Luftaustrittskanal 10 die Druckluft dem distalen Ende des Endoskops 2 zugeführt werden.

Nach der Figur 10 wird der Finger 52 nicht nur auf die Stirnseite des Betätigungsknopfes 17 aufgelegt, sondern es wird zusätzlich auch eine Kraft auf den Ventilschieber 5 in Längsrichtung ausgeübt, die zu dessen Längsverschiebung entgegen der Kraft der Druckfeder 23 führt. In einer Endstellung des Ventilschiebers 5 gelangt der Dichtring 41 in den Zulaufraum 49 und übernimmt keine Dichtfunktion, so dass Spülflüssigkeit aus dem Spülflüssigkeitszufuhrkanal 7 in den Spülflüssigkeitsaustrittskanal 9 gelangt. Der Steuerraum 51 ist dabei mittels des Dichtrings 42 abgedichtet. Außerdem liegt der distale Teilbereich 46 des Dichtrings 43, der als Sitzdichtring ausgebildet ist, an dem Ventilsitz 56 an. Dadurch wird verhindert, dass die Druckluft weiterhin über die Querbohrung 31 und den Längskanal 30 bis zur Stirnseite 17a des Betätigungsknopfes 17 geführt wird.

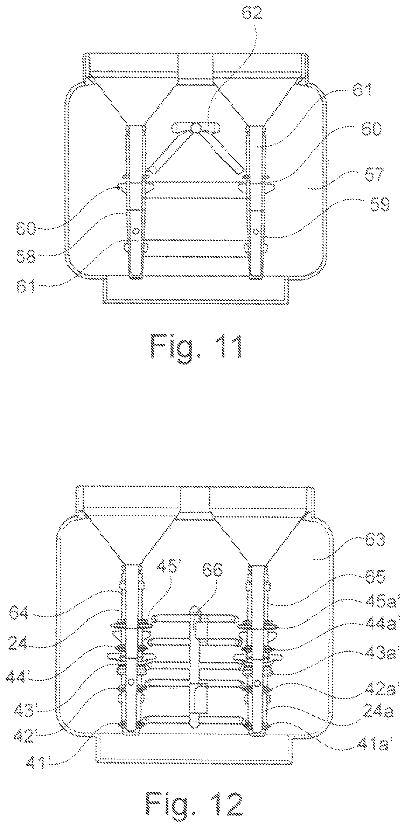

Schließlich zeigt die Figur 11 eine Hälfte einer ersten Spritzgießform 57, mittels welcher der prismenartige Hohlkörper nach den Figuren 4 und 5 hergestellt wird. Die gesamte Spritzgießform 57 wird durch zwei Hälften gebildet. Die vorliegende Spritzgießform 57 weist zwei Kavitäten 58 und 59 auf, so dass in einem Spritzvorgang zwei prismenartige Hohlkolben 24 aus einem Acryl Butadien Styrol (ABS) herstellbar sind. Jede dieser Kavitäten 58 und 59 weist eine Matrize 60 und einen Kern 61 auf. Der flüssige Kunststoff wird den Kavitäten 58 und 59 über ein Angusssystem 62 zugeführt. Nach dem Erstarren der Formlinge, also der beiden Hohlkörper 24 und 24a wird die Spritzgießform 57 getrennt und die beiden Hohlkörper 24, 24a mittels eines nicht näher dargestellten Auswerfers aus einer Auswerferseite der Spritzgießform 57 ausgeworfen.

Aus der Figur 12 geht eine Hälfte einer zweiten Spritzgießform 63 mit zwei Matrizen 64 und 65 hervor, in die die beiden in der ersten Spritzgießform 57 hergestellten Hohlkörper 24 und 24a eingesetzt werden. Die Matrizen 54 und 65 weisen eine negative Kontur 41', 42', 43', 44', und 45' sowie 41a', 42a', 43a', 44a', und 45a' der jeweiligen in den Figuren 2 und 3 gezeigten Dichtringe 41, 42, 43, 44 und 45 auf. Die beiden Hohlkörper 24 und 24a weisen eine Resttemperatur von 60 bis 100°C auf, wenn das thermoplastische Elastomer (TE) über ein Angusssystem 66 in die Matrizen eingespritzt wird. Anschließend findet die Entformung der beiden im Wesentlichen fertigen Ventilschieber statt, die anschließend nur noch mit der Druckfeder 23, dem Haltering 13 und dem Betätigungsknopf 17 versehen werden.

### Bezugszeichenliste

- 1: Handbetätigungsteil von 2
- 2: Endoskop
- 3: Ventileinheit
- 4: Entleerventil
- 5: Ventilschieber
- 5a: proximales Ende von 5
- 6: Aufnahmebohrung
- 6a: Innenmantelfläche von 6
- 7: Spülflüssigkeitszuflusskanal
- 8: Luftzufuhrkanal
- 9: Spülflüssigkeitsaustrittskanal
- 10: Luftaustrittskanal
- 11: Druckluftquelle
- 11a: Druckluftleitung
- 12: Spülflüssigkeitsvorratsbehälter
- 13: Haltering
- 14: elastische Muffel
- 15: Gehäuse von 1
- 16: Bund von 15
- 17: Betätigungsknopf
- 17a: muldenartige Stirnseite von 17
- 18: Entleerkanal
- 19: Sammelkanal
- 20: Sammelreservoir
- 21: Haltering
- 22: Druckknopf
- 22a: Druckfeder von 4
- 23: Druckfeder von 3
- 24: prismenartiger Hohlkörper
- 24a: prismenartiger Hohlkörper
- 25: erster Abschnitt der Außenwand
- 25a: endseitige Nut in 25
- 25b: Rippen von 25
- 26: Längsschlitze in 22
- 27: zweiter Abschnitt der Außenwand
- 28: dritter Abschnitt der Außenwand
- 29: stirnseitige Wand
- 30: Längskanal
- 31: Querbohrung
- 32: erste Kufen
- 33: Anfasung von 32
- 34: Ende von 32
- 35: zweite Kufen
- 36: Anfasung von 35
- 37: Ende von 35
- 38: ringförmiger Vorsprung
- 39: Nabe von 13
- 40: Außenmantelfläche von 24
- 41: Dichtring
- 41a: Matrize für Dichtring
- 41a': Matrize für Dichtring
- 42: Dichtring
- 42a: Matrize für Dichtring
- 42a': Matrize für Dichtring
- 43: Dichtring
- 43a: Matrize für Dichtring
- 43a': Matrize für Dichtring
- 44: Dichtring
- 44a: Matrize für Dichtring
- 44a': Matrize für Dichtring
- 45: Dichtring
- 45a: Matrize für Dichtring
- 45a': Matrize für Dichtring
- 46: distaler als Sitzdichtring ausgebildeter Teilbereich von 43
- 47: proximaler als Nutring ausgebildeter Teilbereich von 43
- 48: Ventilgehäuse von 3
- 49: Zulaufraum
- 50: Dichtbereich
- 51: Steuerraum
- 52: Dichtbereich
- 53: radiale Erweiterung
- 54: Dichtbereich
- 55: Dichtbereich
- 56: Ventilsitz
- 57: erste Spritzgießform
- 58: Kavität von 57
- 59: Kavität von 57
- 60: Matrize
- 61: Kern
- 62: Angusssystem
- 63: zweite Spritzgießform
- 64: Matrize von 63
- 65: Matrize von 63
- 66: Angusssystem

## Patentansprüche

1. Ventilschieber (5) für eine als Insufflations- und Spülventil dienende Ventileinheit (3) eines medizinischen Endoskops (2), der mit einem distalen Steuerungsabschnitt in eine mit Luftzufuhr- (8) und Luftaustrittskanälen (10) sowie Spülflüssigkeitszufuhr- (7) und Spülflüssigkeitsaustrittskanälen (9) verbundene, zumindest mittelbar in einem Handbetätigungsteil (1) des Endoskops (2) ausgebildete Aufnahmebohrung (6) einsetzbar ist, wobei der Ventilschieber (5) einen proximalen Betätigungsabschnitt (5a) aufweist, der durch einen Haltering (13) hindurchgeführt sowie mit einem Betätigungsknopf (17) versehen ist, wobei der Betätigungsknopf (17) über eine Druckfeder (23) einerseits am Haltering (13) abgestützt ist und andererseits ein ringförmiger Vorsprung (38) des Ventilschiebers (5) als in axialer Richtung wirkender Anschlag mit dem Haltering (13) zusammenwirkt, und wobei am distalen Steuerungsabschnitt des Ventilschiebers (5) Dichtringe (41, 42, 43, 44 und 45) vorgesehen sind, die radial über den Ventilschieber (5) vorstehen und vorgesehen sind, um, gleitend an einer Innenmantelfläche (6a) der Aufnahmebohrung (6) geführt, zumindest einen Steuerraum (51) der Ventileinheit (3) abzudichten, **dadurch gekennzeichnet, dass** eine Außenmantelfläche (40) des distalen Steuerungsabschnittes (27, 28), abgesehen von an dieser eventuell vorgesehenen radialen Führungsabschnitten (32, 35) sowie dem Vorsprung (38), glattwandig ausgebildet ist und dass die Dichtringe (41, 42, 43, 44 und 45) stoffschlüssig mit der glattwandigen Außenmantelfläche (40) verbunden sind.

2. Ventileinheit (3) eines medizinischen Endoskops (2), die als Insufflationsventil und Spülventil mit einem gemeinsamen Ventilschieber (5) dient, der mit einem distalen Steuerungsabschnitt (27, 28) verschiebbar in einer Aufnahmebohrung (6) eines Ventilgehäuses (48) angeordnet ist, wobei in die Aufnahmebohrung (6) ein mit einer Druckluftquelle (11) verbundener Luftzufuhrkanal (8) und ein mit einer Flüssigkeitsversorgung (12) verbundener Spülflüssigkeitszufuhrkanal (7) münden sowie von dieser ein Luftaustrittskanal (10) und ein Spülflüssigkeitsaustrittskanal (9) ausgehen, die zu einem distalen Ende des Endoskops (2) führen, wobei der Ventilschieber (5) mit einem proximalen, axial über einen den Ventilschieber (5) umschließenden Haltering (21) vorstehenden Betätigungsabschnitt (5a) versehen ist, welcher an seinem Ende mit einem Betätigungsknopf (17) verbunden ist, wobei der Betätigungsknopf (17) einerseits am Haltering (21) über eine Druckfeder (23) abgestützt ist und andererseits mit dem Haltering (21) ein ringförmiger Vorsprung (38) des Ventilschiebers (5) als in axialer Richtung wirkender Anschlag zusammenwirkt, und wobei am distalen Steuerungsabschnitt (27, 28) des Ventilschiebers (5) Dichtringe (42, 43, 44 und 45) vorgesehen sind, die radial über den Ventilschieber (5) vorstehen und gleitend an einer Innenmantelfläche (6a) der Aufnahmebohrung (6) geführt sind, um Steuerräume (49, 51) der Ventileinheit (5) zueinander abzudichten, **dadurch gekennzeichnet, dass** zumindest eine Außenmantelfläche (40) im Bereich des distalen Steuerungsabschnittes (27, 28), abgesehen von an dieser eventuell vorgesehenen radial vorstehenden Führungsabschnitten (32, 35), glattwandig ausgebildet ist und dass die Dichtringe (42, 43, 44 und 45) stoffschlüssig mit der glattwandigen Außenmantelfläche (40) verbunden sind.

3. Ventilschieber oder Ventileinheit nach einem der Patentansprüche 1 oder 2, **dadurch gekennzeichnet, dass** der Ventilschieber (5) als prismenartiger Hohlkörper (24, 24a) mit einem kreisringförmigen Querschnitt ausgebildet ist, in dem zur Leitung der Druckluft ein Längskanal (30) vorgesehen ist.

4. Ventilschieber oder Ventileinheit nach einem der Patentansprüche 1 oder 2, **dadurch gekennzeichnet, dass** der Hohlkörper (24, 24a) aus Acryl Butadien Styrol und die Dichtringe (42, 43, 44 und 45) aus einem thermoplastischen Elastomer hergestellt sind.

5. Ventilschieber oder Ventileinheit nach Patentanspruche 3, **dadurch gekennzeichnet, dass** der Hohlkörper (24, 24a) zumindest im distalen Bereich (27, 28) des Steuerungsabschnittes des Ventilschiebers (5) kegelstumpfförmig ausgebildet ist.

6. Ventilschieber oder Ventileinheit nach Patentanspruch 3, **dadurch gekennzeichnet, dass** der prismenartige Hohlkörper (24, 24a), abgesehen von den Bereichen der Führungsabschnitte (32, 35), des ringförmigen Vorsprunges, proximaler Rippen (25b) und einer endseitigen Nut (25a) mit im Wesentlichen gleicher Wandstärke ausgebildet ist.

7. Ventilschieber oder Ventileinheit nach einem der Patentansprüche 1 oder 2, **dadurch gekennzeichnet, dass** die Führungsabschnitte als am Umfang des Hohlkörpers (24, 24a) gleichmäßig verteilte Kufen (32 und 35) ausgeführt sind, die durch sich vom Umfang aus radial erstreckende Ansätze gebildet werden.

8. Ventilschieber oder Ventileinheit nach Patentanspruch 3, **dadurch gekennzeichnet, dass** in einem zwischen zwei Dichtringen (42 und 43) liegenden Bereich in der Wandung des Hohlkörpers (24, 24a) zumindest eine mit dem Längskanal (30) kommunizierende Querbohrung (31) vorgesehen ist.

9. Ventileinheit nach Patentanspruch 2, **dadurch gekennzeichnet, dass** der Luftzufuhrkanal (8) permanent mit einem stirnseitig des Betätigungsabschnitts mündenden Steuerkanal verbunden ist.

10. Ventileinheit nach einem der Patentansprüche 1 oder 2, **dadurch gekennzeichnet, dass** ein zur Steuerung des Druckluftstromes dienender Dichtring (43), der in beiden Schaltstellungen des Ventilschiebers (5) eine Lage zwischen dem Luftzufuhrkanal (8) und dem Luftaustrittskanal (10) einnimmt als Rückschlagventil wirkt und nur einen Druckluftstrom vom Luftzufuhrkanal (8) zum Luftaustrittskanal (10) freigibt.

11. Ventileinheit nach einem der Patentansprüche 1 oder 2, **dadurch gekennzeichnet, dass** die den Ventilschieber (5) aufnehmende Aufnahmebohrung (6) in ihrem ein distales Ende bildenden axialen Abschnitt, der an den Spülflüssigkeitszuflusskanal (7) angeschlossen ist, gegenüber einem sich daran in proximaler Richtung anschließenden Dichtbereich (50) radial erweitert ist.

12. Verfahren zur Herstellung eines Ventilschiebers (5) für eine aus einem Insufflations- und einem Spülventil bestehende Ventileinheit (3) eines medizinischen Endoskops (2), der einen proximalen Betätigungsabschnitt (25) aufweist, wobei an einem distalen Steuerungsabschnitt (27, 28) des Ventilschiebers (5) Dichtringe (42, 43, 44 und 45) vorgesehen sind, **dadurch gekennzeichnet, dass** der als prismenartiger Hohlkörper (24, 24a) mit kreisringförmigem Querschnitt ausgebildete Ventilschieber (5) in einem ersten Arbeitsschritt in einer ersten, einen Kern (61) aufnehmenden Spritzgießform (57) mit einer glattwandig ausgebildeten Außenmantelfläche (40), an der radiale Führungsabschnitten (32, 35) vorgesehen sind, herstellt wird, und dass dieser prismenartige Hohlkörper (24, 24a) im noch nicht erkalteten Zustand in einem zweiten Arbeitsschritt in eine zweite, Außenkonturen der Dichtringe (42, 43, 44 und 45) aufweisende Spritzgießform eingesetzt wird und die Dichtringe (42, 43, 44 und 45) an die glattwandige Außenmantelfläche (40) des Hohlkörpers (24, 24a) angespritzt werden.

13. Verfahren nach Patentanspruch 12, **dadurch gekennzeichnet, dass** die Schmelzpunkte der für den Hohlkörper (24, 24a) und die Dichtringe (42, 43, 44 und 45) verwendeten Werkstoffe nahe beieinander liegen.

14. Verfahren nach Patentanspruch 13, **dadurch gekennzeichnet, dass** als Werkstoff des Hohlkörpers Acryl Butadien Styrol und als Werkstoff der Dichtringe ein thermoplastisches Elastomer verwendet werden.

15. Verfahren nach Patentanspruch 12, **dadurch gekennzeichnet, dass** der Hohlkörper (24, 24a) beim Anspritzen der Dichtringe (42, 43, 44 und 45) eine Oberflächentemperatur von 60 - 100° aufweist.

## Claims

1. Valve slide (5) for a valve unit (3), serving as an insufflation and purging valve, of a medical endoscope (2), which valve slide (5) can be inserted by way of a distal control section into a receiving bore (6) which is configured at least indirectly in a manual actuation part (1) of the endoscope (2) and is connected to air inlet channels (8) and air outlet channels (10) and to purging liquid inflow channels (7) and purging liquid outlet channels (9), the valve slide (5) having a proximal actuating section (5a) which is guided through a retaining ring (13) and is provided with an actuating button (17), the actuating button (17) being supported firstly on the retaining ring (13) via a compression spring (23), and secondly an annular projection (38) of the valve slide (5) interacting as a stop which acts in the axial direction with the retaining ring (13), and sealing rings (41, 42, 43, 44 and 45) being provided on the distal control section of the valve slide (5), which sealing rings (41, 42, 43, 44 and 45) project radially beyond the valve slide (5) and are provided to seal at least one control space (51) of the valve unit (3) in a manner which slides on an inner circumferential face (6a) of the receiving bore (6), **characterized in that** an outer circumferential face (40) of the distal control section (27, 28) is of smooth-walled configuration, apart from the projection (38) and radial guide sections (32, 35) which are possibly provided on said outer circumferential face (40), and **in that** the sealing rings (41, 42, 43, 44 and 45) are connected in an integrally joined manner to the smooth-walled outer circumferential face (40).

2. Valve unit (3) of a medical endoscope (2), which valve unit (3) serves as an insufflation valve and a purging valve with a common valve slide (5) which is arranged by way of a distal control section (27, 28) in a receiving bore (6) of a valve housing (48) such that it can be displaced, an air inlet channel (8) which is connected to a compressed air source (11) and a purging liquid inflow channel (7) which is connected to a liquid supply (12) opening into the receiving bore (6), and an air outlet channel (10) and a purging liquid outlet channel (9) emanating from said receiving bore (6), which channels lead to a distal end of the endoscope (2), the valve slide (5) being provided with a proximal actuating section (5a) which projects axially beyond a retaining ring (21) which encloses the valve slide (5), which actuating section (5a) is connected at its end to an actuating button (17), the actuating button (17) being supported firstly on the retaining ring (21) via a compression spring (23), and secondly an annular projection (38) of the valve slide (5) interacting as a stop which acts in the axial direction with the retaining ring (21), and sealing rings (42, 43, 44 and 45) being provided on the distal control section (27, 28) of the valve slide (5), which sealing rings (42, 43, 44 and 45) project radially beyond the valve slide (5) and are guided in a sliding manner on an inner circumferential face (6a) of the receiving bore (6), in order to seal control spaces (49, 51) of the valve unit (5) with respect to one another, **characterized in that** at least one outer circumferential face (40) is of smooth-walled configuration in the region of the distal control section (27, 28), apart from radially projecting guide sections (32, 35) which are possibly provided on said outer circumferential face (40), and **in that** the sealing rings (42, 43, 44 and 45) are connected in an integrally joined manner to the smooth-walled outer circumferential face (40) .

3. Valve slide or valve unit according to either of Patent Claims 1 and 2, **characterized in that** the valve slide (5) is configured as a prism-like hollow body (24, 24a) with a circularly annular cross section, in which a longitudinal channel (30) is provided for conducting the compressed air.

4. Valve slide or valve unit according to either of Patent Claims 1 and 2, **characterized in that** the hollow body (24, 24a) is produced from acrylonitrile butadiene styrene, and the sealing rings (42, 43, 44 and 45) are produced from a thermoplastic elastomer.

5. Valve slide or valve unit according to Patent Claim 3, **characterized in that** the hollow body (24, 24a) is of frustoconical configuration at least in the distal region (27, 28) of the control section of the valve slide (5).

6. Valve slide or valve unit according to Patent Claim 3, **characterized in that** the prism-like hollow body (24, 24a) is configured with a substantially identical wall thickness, apart from the regions of the guide sections (32, 35), of the annular projection, proximal ribs (25b) and an end-side groove (25a).

7. Valve slide or valve unit according to either of Patent Claims 1 and 2, **characterized in that** the guide sections are configured as runners (32 and 35) which are distributed uniformly on the circumference of the hollow body (24, 24a) and are formed by way of attachments which extend radially from the circumference.

8. Valve slide or valve unit according to Patent Claim 3, **characterized in that** at least one transverse bore (31) which communicates with the longitudinal channel (30) is provided in the wall of the hollow body (24, 24a) in a region which lies between two sealing rings (42 and 43) .

9. Valve unit according to Patent Claim 2, **characterized in that** the air inlet channel (8) is connected permanently to a control channel which opens on the end side of the actuating section.

10. Valve unit according to either of Patent Claims 1 and 2, **characterized in that** a sealing ring (43) which serves to control the compressed air stream and assumes a position between the air inlet channel (8) and the air outlet channel (10) in the two switching positions of the valve slide (5) acts as a check valve and releases only a compressed air stream from the air inlet channel (8) to the air outlet channel (10).

11. Valve unit according to either of Patent Claims 1 and 2, **characterized in that**, in its axial section which forms a distal end and is connected to the purging liquid inflow channel (7), the receiving bore (6) which receives the valve slide (5) is widened radially with respect to a sealing region (50) which adjoins it in the proximal direction.

12. Method for producing a valve slide (5) for a valve unit (3), consisting of an insufflation valve and a purging valve, of a medical endoscope (2), which valve slide (5) has a proximal actuating section (25), sealing rings (42, 43, 44 and 45) being provided on a distal control section (27, 28) of the valve slide (5), **characterized in that** the valve slide (5) which is configured as a prism-like hollow body (24, 24a) with a circularly annular cross section is produced in a first working step in a first injection mold (57) which receives a core (61) and has an outer circumferential face (40) of smooth-walled configuration, on which radial guide sections (32, 35) are provided, and **in that** said prism-like hollow body (24, 24a) is inserted in the state, in which it has not yet cooled, in a second working step into a second injection mold which has outer contours of the sealing rings (42, 43, 44 and 45), and the sealing rings (42, 43, 44 and 45) are molded onto the smooth-walled outer circumferential face (40) of the hollow body (24, 24a).

13. Method according to Patent Claim 12, **characterized in that** the melting points of the materials which are used for the hollow body (24, 24a) and the sealing rings (42, 43, 44 and 45) lie close to one another.

14. Method according to Patent Claim 13, **characterized in that** acrylonitrile butadiene styrene is used as material of the hollow body, and a thermoplastic elastomer is used as material of the sealing rings.

15. Method according to Patent Claim 12, **characterized in that** the hollow body (24, 24a) has a surface temperature of 60-100° in the case of molding on of the sealing rings (42, 43, 44 and 45).

## Revendications

1. Tiroir de soupape (5) destiné à une unité de soupape (3) servant de soupape d'insufflation et de vidange d'un endoscope médical (2), lequel tiroir peut être inséré par une section de commande distale dans un alésage de réception (6) connecté à des canaux d'amenée d'air (8) et des canaux de sortie d'air (10) ainsi qu'à des canaux d'amenée de liquide de vidange (7) et des canaux de sortie de liquide de vidange (9) et réalisé au moins indirectement dans une partie d'actionnement manuel (1) de l'endoscope (2), le tiroir de soupape (5) comprenant une section d'actionnement proximale (5a) qui est guidée à travers une bague de retenue (13) et est dotée d'un bouton d'actionnement (17), le bouton d'actionnement (17) étant supporté d'une part sur la bague de retenue (13) par le biais d'un ressort de compression (23) et d'autre part une saillie (38) annulaire du tiroir de soupape (5) coopérant, en tant que butée agissant dans la direction axiale, avec la bague de retenue (13), et des bagues d'étanchéité (41, 42, 43, 44 et 45) étant prévues sur la section de commande distale du tiroir de soupape (5), lesquelles font saillie radialement au-delà du tiroir de soupape (5) et sont prévues pour rendre étanche au moins un espace de commande (51) de l'unité de soupape (3) de manière guidée de façon glissante sur une surface d'enveloppe intérieure (6a) de l'alésage de réception (6), **caractérisé en ce qu'**une surface d'enveloppe extérieure (40) de la section de commande (27, 28) distale, mis à part des sections de guidage (32, 35) radiales éventuellement prévues sur celle-ci ainsi que la saillie (38), est réalisée avec une paroi lisse, et **en ce que** les bagues d'étanchéité (41, 42, 43, 44 et 45) sont connectées par liaison de matière à la surface d'enveloppe extérieure (40) à paroi lisse.

2. Unité de soupape (3) d'un endoscope médical (2), qui sert de soupape d'insufflation et de soupape de vidange comportant un tiroir de soupape (5) commun, lequel est disposé dans un alésage de réception (6) d'un carter de soupape (48) de manière déplaçable par une section de commande (27, 28) distale, un canal d'amenée d'air (8) connecté à une source d'air comprimé (11) et un canal d'amenée de liquide de vidange (7) connecté à une alimentation en liquide (12) débouchant dans l'alésage de réception (6) et un canal de sortie d'air (10) et un canal de sortie de liquide de vidange (9) partant de cet alésage de réception, lesquels canaux mènent à une extrémité distale de l'endoscope (2), le tiroir de soupape (5) étant doté d'une section d'actionnement (5a) proximale faisant saillie axialement au-delà d'une bague de retenue (21) entourant le tiroir de soupape (5), laquelle section d'actionnement est connectée, à son extrémité, à un bouton d'actionnement (17), le bouton d'actionnement (17) étant supporté d'une part sur la bague de retenue (21) par le biais d'un ressort de compression (23) et d'autre part une saillie (38) annulaire du tiroir de soupape (5) coopérant, en tant que butée agissant dans la direction axiale, avec la bague de retenue (21), et des bagues d'étanchéité (41, 42, 43, 44 et 45) étant prévues sur la section de commande (27, 28) distale du tiroir de soupape (5), lesquelles font saillie radialement au-delà du tiroir de soupape (5) et sont guidées de façon glissante sur une surface d'enveloppe intérieure (6a) de l'alésage de réception (6), pour rendre étanche les uns par rapport aux autres des espaces de commande (49, 51) de l'unité de soupape (5), **caractérisée en ce qu'**au moins une surface d'enveloppe extérieure (40) dans la région de la section de commande (27, 28) distale, mis à part des sections de guidage (32, 35) faisant saillie radialement éventuellement prévues sur celle-ci, est réalisée avec une paroi lisse, et **en ce que** les bagues d'étanchéité (41, 42, 43, 44 et 45) sont connectées par liaison de matière à la surface d'enveloppe extérieure (40) à paroi lisse.

3. Tiroir de soupape ou unité de soupape selon l'une des revendications 1 ou 2, caractérisé (e) en ce que le tiroir de soupape (5) est réalisé en tant que corps creux (24, 24a) prismatique présentant une section transversale en forme d'anneau circulaire, corps creux dans lequel un canal longitudinal (30) est prévu pour le guidage de l'air comprimé.

4. Tiroir de soupape ou unité de soupape selon l'une des revendications 1 ou 2, caractérisé (e) en ce que le corps creux (24, 24a) est fabriqué à partir d'acrylbutadiènestyrène et les bagues d'étanchéité (42, 43, 44 et 45) sont fabriquées à partir d'un élastomère thermoplastique.

5. Tiroir de soupape ou unité de soupape selon la revendication 3, caractérisé(e) en ce que le corps creux (24, 24a) est réalisé sous forme tronconique au moins dans la région distale (27, 28) de la section de commande du tiroir de soupape (5).

6. Tiroir de soupape ou unité de soupape selon la revendication 3, caractérisé(e) en ce que le corps creux (24, 24a) prismatique est réalisé avec une épaisseur de paroi sensiblement identique, mis à part les régions des sections de guidage (32, 35), de la saillie annulaire, de nervures proximales (25b) et d'une rainure (25a) du côté de l'extrémité.

7. Tiroir de soupape ou unité de soupape selon l'une des revendications 1 ou 2, caractérisé(e) en ce que les sections de guidage sont configurées en tant que patins (32 et 35) répartis uniformément sur la périphérie du corps creux (24, 24a), lesquels sont formés par des rallonges s'étendant radialement à partir de la périphérie.

8. Tiroir de soupape ou unité de soupape selon la revendication 3, caractérisé(e) en ce qu'au moins un alésage transversal (31) communiquant avec le canal longitudinal (30) est prévu dans une région située entre deux bagues d'étanchéité (42 et 43) dans la paroi du corps creux (24, 24a).

9. Unité de soupape selon la revendication 2, **caractérisée en ce que** le canal d'amené d'air (8) est connecté en permanence à un canal de commande débouchant sur le côté frontal de la section d'actionnement.

10. Unité de soupape selon l'une des revendications 1 ou 2, **caractérisée en ce qu'**une bague d'étanchéité (43) servant à la commande du flux d'air comprimé, laquelle adopte une position entre le canal d'amené d'air (8) et le canal de sortie d'air (10) dans les deux positions de commutation du tiroir de soupape (5), agit comme soupape anti-retour et ne libère qu'un flux d'air comprimé du canal d'amené d'air (8) au canal de sortie d'air (10).

11. Unité de soupape selon l'une des revendications 1 ou 2, **caractérisée en ce que** l'alésage de réception (6) recevant le tiroir de soupape (5), dans sa section axiale qui forme une extrémité distale et qui est raccordée au canal d'amené de liquide de vidange (7), est élargi radialement par rapport à une région d'étanchéité (50) s'y raccordant dans la direction proximale.

12. Procédé de fabrication d'un tiroir de soupape (5) destiné à une unité de soupape (3) constituée d'une soupape d'insufflation et d'une soupape de vidange d'un endoscope médical (2), lequel tiroir comprend une section d'actionnement (25) proximale, des bagues d'étanchéité (42, 43, 44 et 45) étant prévues sur une section de commande (27, 28) distale du tiroir de soupape (5), **caractérisé en ce que** le tiroir de soupape (5) réalisé en tant que corps creux (24, 24a) prismatique présentant une section transversale en forme d'anneau circulaire est, dans une première étape de travail, fabriqué dans un premier moule de moulage par injection (57) recevant un noyau (61) et présentant une surface d'enveloppe extérieure (40) réalisée avec une paroi lisse, surface sur laquelle des sections de guidage (32, 35) radiales sont prévues, et **en ce que** ce corps creux (24, 24a) prismatique est inséré, dans l'état non encore refroidi, dans une deuxième étape de travail, dans un deuxième moule de moulage par injection présentant des contours extérieurs des bagues d'étanchéité (42, 43, 44 et 45) et les bagues d'étanchéité (42, 43, 44 et 45) sont moulées par injection sur la surface d'enveloppe extérieure (40) à paroi lisse du corps creux (24, 24a).

13. Procédé selon la revendication 12, **caractérisé en ce que** les points de fusion des matières utilisées pour le corps creux (24, 24a) et les bagues d'étanchéité (42, 43, 44 et 45) sont proches l'un de l'autre.

14. Procédé selon la revendication 13, **caractérisé en ce que** de l'acrylbutadiènestyrène est utilisé comme matière du corps creux et un élastomère thermoplastique est utilisé comme matière des bagues d'étanchéité.

15. Procédé selon la revendication 12, **caractérisé en ce que** le corps creux (24, 24a) présente une température de surface de 60 à 100° lors du moulage par injection des bagues d'étanchéité (42, 43, 44 et 45).
